(19) Europäisches Patentamt
European Patent Office
Office européen des brevets

(11) **EP 3 748 342 B1**

(12) **EUROPEAN PATENT SPECIFICATION**

(45) Date of publication and mention
of the grant of the patent:
**03.05.2023 Bulletin 2023/18**

(21) Application number: **19178679.7**

(22) Date of filing: **06.06.2019**

(51) International Patent Classification (IPC):
*D01H 13/16* (2006.01)     *G01N 21/89* (2006.01)
*G01N 33/36* (2006.01)

(52) Cooperative Patent Classification (CPC):
**D01H 13/165; G01N 21/8915; G01N 33/365**

(54) **OPTICAL SENSOR FOR MEASURING A PROPERTY OF AN ELONGATE TEXTILE BODY IN A UNIFORM OPTICAL FIELD**

OPTISCHER SENSOR ZUR MESSUNG EINER EIGENSCHAFT EINES LÄNGLICHEN TEXTILKÖRPERS IN EINEM GLEICHMÄSSIGEN OPTISCHEN FELD

CAPTEUR OPTIQUE POUR MESURER UNE PROPRIÉTÉ D'UN CORPS TEXTILE ALLONGÉ DANS UN CHAMP OPTIQUE UNIFORME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.12.2020 Bulletin 2020/50**

(73) Proprietor: **Gebrüder Loepfe AG**
**8623 Wetzikon (CH)**

(72) Inventors:
• **Schlatter, Adrian**
**8046 Zürich (CH)**
• **Griesshammer, Markus**
**8037 Zürich (CH)**

(74) Representative: **Sutter, Kurt**
**E. Blum & Co. AG**
**Vorderberg 11**
**8044 Zürich (CH)**

(56) References cited:
**EP-A1- 1 643 246        EP-B1- 0 572 592**
**US-A- 4 716 942        US-A- 4 805 671**
**US-A1- 2011 303 848**

Remarks:
The file contains technical information submitted after the application was filed and not included in this specification

EP 3 748 342 B1

**Description**

Technical Field

The invention relates to a sensor for measuring at least one property of an elongate body, in particular an elongate textile body, such as a yarn.

Background Art

This type of sensor can e.g. be used for measuring the diameter of a yarn, wire, or fiber. The elongate body is guided through a measuring region and viewed in transmission therein.
The sensor comprises a light source, a collimator, a measurement slot and a detector. The collimator has an output optical axis extending through the measuring region. It converts the typically divergent light from the light source into substantially parallel light along said optical axis. An example for such a sensor is described in EP 2827127. The light source, the lens, and the light sensor are arranged along one common optical axis.
US 47616942 describes a sensor with a transparent element having a curved mirror surface for generating a collimated light field in a measuring region.

Disclosure of the Invention

Having realized that a stringent coaxial arrangement of the components as shown in the prior art makes the design, alignment and assembly of a sensor difficult and expensive, the problem solved by the present invention is to provide a sensor with a more flexible design.
This problem is solved by sensor of claim 1. Accordingly, the sensor comprises at least the following components:

- A measuring region: This is the region of the sensor designed for receiving the elongate body.
- A divergent, light source having anisotropic far-field intensity with a main emission direction: This light source can e.g. be a LED. In this context, the term "main emission direction" is the weighted average of all emission directions of the light source, where each emission direction is weighted by its radiant intensity.

- A collimator arranged in a light path between the light source and the measuring region: The collimator has an input optical axis intersecting the center of the light source. It further has an output optical axis. It projects light from said light source on the input optical axis onto the output optical axis. Its function is to generate collimated light in the measuring region, i.e. light that is substantially parallel as defined below. The output optical axis intersects the center of the measuring region.
- A light detector: This detector receives the light emitted by the collimator and transmitted through the measuring region.

According to the invention, the input optical axis and the output optical axis of the collimator are non-parallel and the main emission direction of the light source is non-parallel to the input optical axis.
Arranging the input optical axis and the output optical axis in a non-parallel manner allows "folding" the optical axis of the device.
Such folding, however, typically leads to a non-uniform distribution of the irradiance in the measuring region. As explained in more detail below, such a geometry renders the collimator's transmission function non-symmetric in the folding plane of the system. Hence, the invention suggests arranging the main emission direction of the light source not along the input optical axis of the collimator. This will reduce the irradiance in the center of the measuring region, but it allows exploiting the asymmetry of the radiant intensity of the light source at directions away from its main emission direction for compensating, at least in part, the asymmetry of the transmission function of the collimator.
In an advantageous embodiment, the following condition should be met at least in the plane of input and output optical axes:
Assume that a light beam emitted from the center of the light source having an angle $\varphi$ in respect to the main emission direction of the light source transverses the measuring region at a location $x, y$, with $x, y$ being a coordinates perpendicular to the output optical axis and having a value $x0, y0$ at the location of the output optical axis. If $I(x, y)$ is the irradiance at the location $x, y$ in the measuring region, $I(x, y)$ is given by the transmission function $T(x, y)$ of the collimator as well as by the radiant intensity $i(\varphi(x,y), \vartheta(x,y))$ of the light source as

$$I(x, y) \sim T(x, y) \cdot i(\varphi(x, y), \vartheta(x, y))$$

In that case, for *x = x0 and y = y0* (i.e. in the center of the measuring region) and as explained below, the transmission function *T(x, y)* and the radiant intensity *i(φ(x, y))* should fulfil one of the two following conditions:

$$\mathrm{d}T(x,y)/\mathrm{d}y > 0 \text{ and } \mathrm{d}i(\varphi(x, y), \vartheta(x, y))/\mathrm{d}y < 0$$

or

$$\mathrm{d}T(x,y)/\mathrm{d}y < 0 \text{ and } \mathrm{d}i(\varphi(x, y), \vartheta(x, y))/\mathrm{d}y > 0$$

In other words, the changes of radiant intensity *i(φx, y))* of the light source and the transmission function *T(x,y)* of the collimator for small Δ*y = y - y*0 advantageously have opposite signs. This allows to compensate the effect of the non-homogeneous transmission function *T(x,y)* at least in part.

The collimator comprises a collimation mirror for deflecting the light from the light source.

In a particularly advantageous embodiment, the collimation mirror can have parabolic or paraboloid shape (as defined below) at least around the point where the input optical axis intersects with the mirror.

Further, the sensor comprises a first transparent element (in the sense that it is transparent for the light from the light source) having an input side, an output side, and a mirror surface. Light from the light source enters the first transparent element through an input surface at the input side, is reflected at the mirror surface, and exits the transparent element through an output surface at the output side to enter said measuring region. The input surface and the output surface are arranged transversally to each other. Thus, the first transparent element forms the collimator.

To reduce refraction, the input surface is located in a concave recess of the first transparent element at the input side.

The light source may be mounted to a carrier plate, such as a PCB, arranged at the input side. This carrier plate is arranged transversally to the output side. Advantageously, the angle between the carrier plate and the output surface is between 70° and 110°.

Advantageously, the main optical axis of the light source extends perpendicular to the carrier plate, e.g. within an accuracy of +/- 5°, which simplifies the mutual mechanical arrangement of the components.

Advantageously, the detector is arranged at a distance from the output optical axis of the collimator, which makes it unnecessary to mount any electronic components at the location of the optical axis, thereby making the design more flexible.

In this case, the detector and the light source may e.g. both be mounted to a common carrier plate, in particular to a flat common carrier plate, such as a common PCB. This simplifies the mounting of the components.

Advantageously, the sensor further comprises a detector mirror receiving light from the measuring region and deflecting it towards the detector. This is simple embodiment allowing to deflect the light away from the output optical axis towards the detector.

The sensor is particularly suited for measuring a diameter of the elongate body. In one advantageous application, the sensor is used in a yarn clearer.

Other advantageous embodiments are listed in the dependent claims as well as in the description below.

Brief Description of the Drawings

The invention will be better understood and objects other than those set forth above will become apparent from the following detailed description thereof. Such description refers to the annexed drawings, wherein:

    Fig. 1 shows a view of a sensor for performing measurements on an elongate body,
    Fig. 2 shows a sectional view through the sensor of Fig. 1 in the plane of input- and output optical axes,
    Fig. 3 is a sectional view along plane III-III of Fig. 2,
    Fig. 4 illustrates the geometry of light deflection in the sensor,
    Fig. 5 illustrates the reflection from a parabolic mirror with an isotropic light source at its focal point,
    Fig. 6 shows the transmission function *T(r)* of the mirror,
    Fig. 7 shows the radiant intensity of a light source with Lambert emission characteristics, and
    Fig. 8 shows the transmission function *T*, radiant intensity *i*, their product *T·i,* as well as the probability distribution *p* of the position of a yarn, all of them as a function of position r divided by the focal length of a mirror with paraboloid shape.

Modes for Carrying Out the Invention

*Definitions*

A *collimator* is to be understood as a device that converts the divergent light of the light source into a field of substantially parallel light beams in the measuring region.

This is to be understood such that, at least in the plane intersecting the center of the light source and being parallel to said input and output optical axes, the light beams in the measuring region are substantially parallel, such that the shadowing caused by the elongate body is, at its statistically expected positions in the measuring region, practically independent, within the desired measurement accuracy, of the distance of the elongate body from the walls of the measuring region.

Advantageously, the divergence of the light in the measuring region is so small that the light entering it through an area A is spread into no more than an area $A \cdot (1 + e)$ when leaving it, with $e$ being smaller than 0.2, in particular smaller than 0.05. If the measuring region has a height $H$, a length $L$ (along the propagation direction of the elongate body), and a width $W$ (with the width $W$ measured along a direction parallel to the average propagation direction of the light field and perpendicular to the longitudinal axis of the elongate body and the height H along a direction perpendicular to the width $W$ and to the elongate body), the divergence $\alpha$ can e.g. be estimated (in linear approximation) by

$$\alpha \leq \arctan(e \cdot \frac{H \cdot L}{2 \cdot W \cdot (H + L)})$$

If $H \cong L \cong W$, we have, e.g., $\alpha \leq 2.9°$, in particular $\alpha \leq 0.7°$.

Advantageously, the above conditions regarding parallelism of the light in the measuring region not only apply to the light in the plane intersecting the center of the light source and being parallel to said input and output optical axes but also in a plane perpendicular thereto and parallel to the average direction of the light. This allows to increase the measurement resolution and/or to use a larger measuring region and/or a smaller detector arrangement.

It must be noted, though, that the light from a light source of finite size can never be collimated perfectly.

A mirror is *concave* in a region when the lines between any two points on the mirror in the region do not intersect the mirror and, for at least some of the lines, the lines do not touch the mirror except at their end points and remain entirely on the illuminated side of the mirror. Hence, the mirror is curved at least in one direction. It may also be curved at least in two directions.

The expression that the mirror has *parabolic shape* is to be understood such that, in a plane intersecting the center of the light source and being parallel to the input and output optical axes, the mirror extends along a parabola with the center of the light source being in the parabola's focal point, which corresponds to a focal line in three dimensions.

The expression that the mirror has *paraboloid shape* is to be understood such that the mirror follows a paraboloid of rotational symmetry in three dimensions, with the center of the light source being in the paraboloid's focal point.

*Transparent* is understood to designate a *body* that is substantially transparent to the light of the light source in the sense that it absorbs or scatters no more than 20%, in particular no more than 10% of the light from the light source passing through it. A transparent *surface* is understood to designate a surface that is substantially transparent to the light of the light source in the sense that it reflects or scatters no more than 20%, in particular no more than 10% of the light from the light source passing through it

A *transparent element* is a solid, transparent body.

The *measuring region* designates the region where the elongate body is located. Since, in operation, the body's position is not stationary but fluctuates, the width and height of the measurement region (in directions perpendicular to the longitudinal axis of the body) are non-zero and given by the statistical distribution of the body's location. For yarn, the width and height may e.g. be 3 - 4 mm. Advantageously, the measuring region corresponds to the region where the elongated body is, in operation, at least in 90% of the time, in particular at least 99% of the time.

*Device Example*

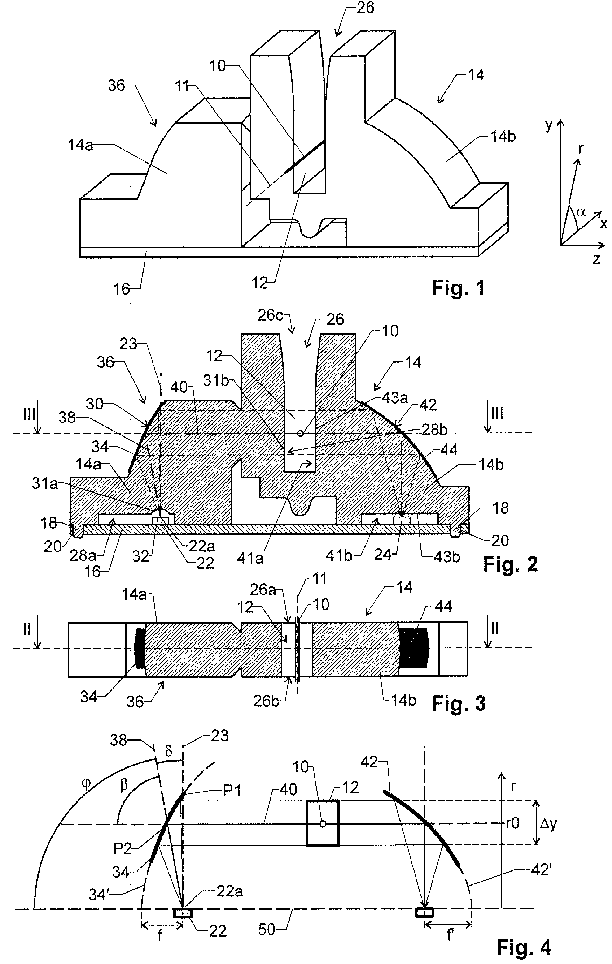

Figs. 1 - 3 show an embodiment of an optical sensor for measuring a property of an elongate object 10 aligned along a longitudinal direction 11 and located in a measuring region 12. In the present example, object 10 is a yarn.

The sensor comprises a transparent body 14 forming a first and a second transparent element 14a, 14b. In the shown embodiment, the two elements 14a, 14b are integrally connected to each other, thereby ensuring a stable, well-defined mutual alignment.

Body 14 can e.g. be cast or molded from a transparent plastic material.

A carrier plate 16 is located adjacent to body 14. It can e.g. be a printed circuit board.

Advantageously, body 14 is mechanically connected to carrier plate 16 in direct manner to ensure accurate mutual alignment. In the illustrated embodiment, projections 18 of body 14 extend through openings 20 of carrier plate 16 with no mechanical clearance (press fit) or a small clearance (comparable to or smaller than a typical extension and positioning accuracy of the light source, e.g. less than 200 μm, in particular less than 100 μm) between them, thus mutually aligning the two parts.

A light source 22 is arranged on carrier plate 16 adjacent to first element 14a, and a light detector 24 is arranged on carrier plate 16 adjacent to second element 14b.

Body 14 forms a slit 26 for receiving elongate body 10. Slit 26 is open at a first side 26a and at a second side 26b (cf. Fig. 3), with first and second side 26a, 26b being transversally, in particular perpendicular, to longitudinal direction 11.

Further, slit 26 may be open at a third side 26c (Fig. 2), parallel to longitudinal direction 11, for inserting elongate body 10 into measuring region 12.

First transparent element 14a has an input side 28a and an output side 28b and a mirror surface 30.

Input side 28a advantageously abuts against carrier plate 16 for mutually positioning the two parts along the direction perpendicular to carrier plate 16.

Light source 22 is a divergent, anisotropic light source, in particular an LED, which may have a flat exit surface. It has a main emission direction 23, which is advantageously perpendicular to carrier plate 16 for easy and defined mounting.

The light from light source 22 enters first transparent element 14a through an input surface 31a at input side 28a, is reflected at mirror surface 30, and exits first transparent element 14a through an output surface 31b at output side 28b. Output side 28b borders measuring region 12.

As mentioned above, input side 28a and output side 28b are transversal to each other.

Advantageously, the angle between carrier plate 16 and output surface 31b is between 70° and 110°, in particular 90°.

First transparent element 14a comprises a concave recess 32 at input side 28a. It is located adjacent to light source 22 and forms the input surface 31a. If there is an air gap (or at least a differently refractive material) between light source 22 and the surface of first transparent element 14a, concave recess 32 reduces refraction of the light from light source 22 as it enters first transparent element 14a.

Advantageously, in order to minimize said refraction, concave recess 32 has circular cross section, at least in the plane intersecting a center 22a of light source 22 and being parallel to the input and output optical axes as defined below, and center 22a of light source 22 is located at the center of this circular cross section.

Mirror surface 30 is advantageously coated with a reflective layer (e.g. of metal) for increasing its optical reflectivity. It forms a collimation mirror 34.

First transparent element 14a forms a collimator 36 as described above. It converts the divergent light field emitted by light source 22 into a substantially parallel light field to be sent through measuring region 12.

Collimator 36 has an input optical axis 38 and an output optical axis 40, which are non-parallel for the reasons mentioned above.

By definition, a light beam entering collimator 36 coincident with input optical axis 38 exits collimator 36 coincident with output optical axis 40. Output optical axis 40 crosses the center of measuring region 12.

Advantageously, center 22a of light source 22 is located on input optical axis 38.

Second transparent element 14b forms a light collector for collecting the light emerging from measuring region 12 and sending it to light detector 24.

Second transparent element 14b again has an input side 41a with an input surface 43a and an output side 41b with an output surface 43b as well as a mirror surface 42.

Mirror surface 42 is advantageously coated with a reflective layer (e.g. of metal) for increasing its optical reflectivity. It forms a detector mirror 44 for deflecting the light from measuring region 12 to detector 24.

Measuring region 12 is located between output surface 31b of first transparent element 14a and input surface 43a of second transparent element 14b.

Advantageously, output surface 31b of first transparent element 14a is parallel, in particular within +/- 10°, to input surface 43a of second transparent element 14b, at least where they border measuring region 12.

Larger angles between output surface 31b and input surface 43a may be achieved by adjusting the mirrors 34 and 44 accordingly.

For the same reason, output optical axis 40 is advantageously perpendicular to output side 28b of first transparent element 14a.

Input side 28a of first transparent element 14a and output side 41b of second transparent element 14b advantageously both abut against carrier plate 16. Advantageously, input side 28a of first transparent element 14a has at least one abutting surface coplanar (i.e. in a common plane) with an abutting surface of output side 41b of second transparent element 14b, in particular within an accuracy of +/- 2 mm or better.

In operation, elongate body 10 is run along longitudinal direction 11 through the measuring region. It is illuminated by light from light source 22, and the light transmitted through measuring region is measured by light detector 24. Variations in thickness of elongate object 10 will change the amount of transmitted light. They can be detected in the signal of light detector 24.

*Collimator Design*

Fig. 4 shows the configuration of some parts of the sensor as well as the paths of the light in the sensor.

Collimator mirror 34 has parabolic or paraboloid shape, as indicated by dotted line 34'. Center 22a of light source 22 is arranged in the focal point of this shape, namely on the shape's axis of symmetry 50 at focal distance f from its apex.

Hence, the light beams emitted by center 22a are reflected from collimator mirror 34 along output optical axis 40, with output optical axis 40 being parallel to axis of symmetry 50. This is true for the light beams in the plane of Fig. 4 if collimator mirror 34 has parabolic shape. And it is true also at locations outside this plane if collimator mirror 34 has paraboloid shape.

In the following, a coordinate system as shown in Fig. 1 is used. Here, direction x designates the direction perpendicular to the plane of the input and output optical axes 38, 40, which (in the shown embodiment) corresponds to the longitudinal direction of elongate body 10. Direction y is perpendicular to optical axis 40 and parallel to the plane of the input and output optical axes 38, 40. Direction z is perpendicular to x and y.

For paraboloid mirrors, which are rotationally symmetric about direction z, we alternatively use a polar coordinate system with radius r and azimuth angle $\alpha$ in plane x, y, also as shown in Fig. 1.

The collimated light beams cross measuring region 12 along direction z.

The position of elongate object 10 in measuring region 12 is not perfectly fixed. In operation, it may fluctuate in the plane of Fig. 4, i.e. in the plane containing input optical axis 38 and output optical axis 40. Such movement should not affect the signal measured by detector 24. For this purpose, the sensor tries to improve the uniformity of the irradiance in measuring region 12, in particular along the coordinate r as shown in Fig. 4. This coordinate r extends perpendicularly to the axis of symmetry 50 of the paraboloid or parabolic shape of collimator mirror 34. We assume that r = 0 at the location of the axis of symmetry 50 and $r = r0$ at the center of measuring region 12.

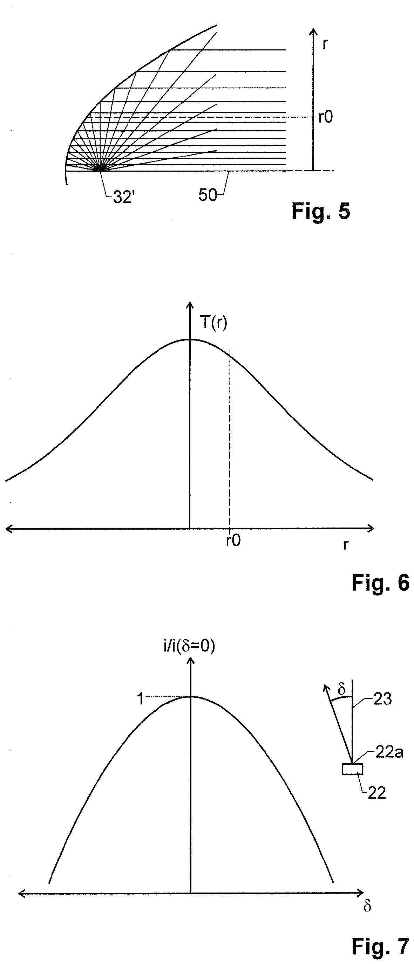

Fig. 5 shows how light from an isotropic light source 32' located in the focal point of a parabolic or paraboloid mirror is reflected and distributed along coordinate r. As can be seen, the irradiance decreases with increasing distance from axis of symmetry 50.

Hence, the transmission function $T(r)$ of the collimator formed by such a collimating mirror decreases with increasing values *of r*. It can be shown that, for paraboloid mirrors and point light sources, the transmission of the collimator as a function of coordinate r can be expressed as follows:

$$T(r) = \frac{1}{f^2} \cdot \frac{16}{(r^2/f^2 + 4)^2} \tag{1}$$

with *f* being the focal distance of the paraboloid mirror.

Fig. 6 shows $T(r)$ as a function *of r*. As can be seen, at a location $r = r0$, $T(r)$ has non-zero slope, i.e. it has lower values for $r > r0$ than for $r < r0$.

Most modern light sources, such as LEDs, fluorescent devices, quantum dot based devices, or laser diodes, are anisotropic. The present design exploits the anisotropy of light source 22.

Many common types of such light sources have a Lambertian emission characteristic or a similar characteristic that has a maximum radiant intensity along main emission direction 23. Fig. 7 plots the radiant intensity $i(\varphi)$ of such a light source 22 with $\varphi$ being the angle in respect to main emission direction 23. This figure assumes, by way of example, that light source 22 has Lambert emission characteristics, i.e.

$$i(\varphi) / i(0) = \cos(\varphi). \tag{2}$$

In a "primitive" approach, the skilled person might be tempted to align main emission direction 23 of light source 22 along input optical axis 38 of collimator 36 or along symmetry axis 50.

As can be seen by combining Figs. 6 and 7, however, this would lead to a strong asymmetry of the irradiance $I(r)$ in measuring region 12 for $r > r0$ and $r < r0$.

Hence, in the present sensor, main emission direction 23 of light source 22 is not chosen to be parallel to optical input axis 38 of collimator 36. Rather, the angle $\delta$ between them is chosen to be non-zero.

In fact, the geometry is chosen such that the intersection point P1 (cf. Fig. 4) between main emission direction 23 and the parabolic or paraboloid shape of mirror 34 is farther away from center 22a of light source 22 than the intersection point P2 of input optical axis 38 and said shape. In that case, the decay of the radiant intensity of light source 22 with increasing values of angle $\delta$ will counteract the slope of the transmission function $T(r)$ of Fig. 6.

This is better understood when analysing the irradiance $I(r)$ in measuring region quantitatively.

First, it must be noted that, for the reflection on a paraboloid, where the center of light source 22 is in the focal point and its main emission direction 23 is perpendicular to axis of symmetry 50, angle $\varphi$, which is the angle between the axis of symmetry of the paraboloid mirror and a light beam as shown in Fig. 4, depends on position $r$ as follows

$$\varphi = \arccos\left(\frac{1-\frac{r^2}{4f^2}}{1+\frac{r^2}{4f^2}}\right) \tag{3}$$

Using Eq. (1) and (3), it can be shown that the irradiance $I(r)$ along y in measuring region 12 has a transmission function $T(r/f)$

$$I(r/f) = \frac{1}{f^2} \cdot T(r/f) \cdot i\big(\varphi(r/f)\big) \tag{4}$$

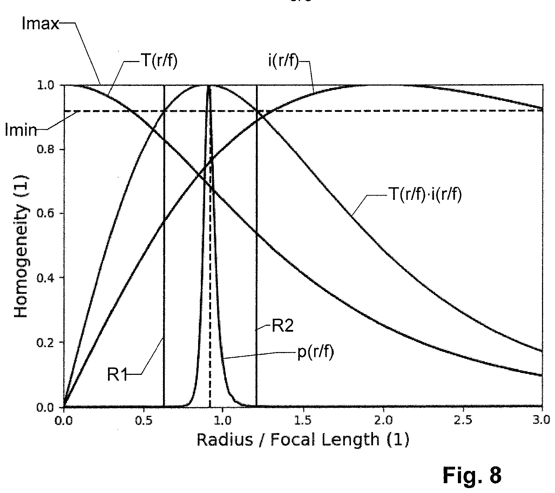

as depicted in Fig. 8. The figure shows plots of the transmission function $T(r/f)$ for $\alpha$=90° and the radiant intensity $i(r/f)$ (for a Lambert light source) as well as their product $T(r/f) \cdot i(r/f)$, with the horizontal axis specifying $r$ in units of $f$. As can be seen, the product, which corresponds to the irradiance $I$ is flattest approximately at $r/f$= 0.9.

This is true for a Lambertian light source. But even if the light source has different emission characteristics, $i(r/f)$ will typically be 0 for $r/f$ = 0 and have a maximum for $r/f$ = 2 if main emission direction 23 of the light source is perpendicular to axis of symmetry 50. Hence, it is advantageous to choose the center $r0$ of measuring region such that the ratio $R = r0/f$ is between 0.5 and 1.4, in particular between 0.7 and 1.1, in particular between 0.8 and 1.0.

As mentioned above, the position of elongate body 10 is not perfectly fixed. Rather, it varies over time, and Fig. 8 shows the probability of elongate body 10 being at a location $r/f$ at any time. As can be seen, in the region where elongate body 10 is present, $T(r/f) \cdot i(r/f)$ is substantially constant.

Advantageously, the deflection angle $\beta$ from input optical axis 38 to output optical axis 40 is smaller than 90° in order to be at a location where the derivatives of the transmission function $T$ and the radiant intensity $i$ in respect to position $r$ have opposite signs. In particular the deflection angle $\alpha$ is smaller than 80° and/or larger than 30°.

Note that Fig. 4 shows the situation in the plane of input optical axis 38 and output optical axis 40. In the direction perpendicular thereto, collimator mirror 34 may e.g. be flat. In that case, however, a uniform irradiance is only achieved in said plane (which is denoted by line II-II of Fig. 3).

To further improve the irradiation homogeneity, collimator mirror 34 is advantageously curved in a direction perpendicular to said plane as well. In particular, collimator mirror 34 advantageously has paraboloid shape as defined above.

It must further be noted that collimator mirror 34 does not necessarily need to have an exactly parabolic or paraboloid shape in a strict mathematical sense. It may have different curvature if small deviations from a perfect collimation of the light beams emitted by center 22a of light source 22 are tolerated, e.g. in order to optimize collimation of light emitted from other locations of light source 22. Also, a deviation from a strictly parabolic or paraboloid shape is allowable if further optical components are introduced for shaping the light beam in the optical collimator, such as input and exit surfaces that are curved to support collimation.

Advantageously, though, collimation mirror 34 is concave, in particular strictly concave (as defined above), at the region around point P2, i.e. where the light from light source 22 coinciding with input optical axis 38 hits it.

Further, it must be noted that collimator 36 may also work without any mirror at all and e.g. rely on a lens design where light source 22 is located off-axis from at least some of the lens surfaces.

In general, for such a collimator, we still have

$$I(x,y) \sim T(x,y) \cdot i\big(\varphi(x,y), \vartheta(x,y)\big) \tag{5}$$

with ~ expressing proportionality, where (x, y) are transverse coordinates in the output of the collimator, and $\varphi(x, y)$ and $\vartheta(x, y)$ are the emission angles of the ray that will hit (x, y) in the output.

Let us review the situation along coordinate *y*, which corresponds to direction r in Fig. 4. The derivative d$I(y)$/d$y$ is given by

$$\frac{\mathrm{d}I(x,y)}{\mathrm{d}y} = \frac{\mathrm{d}}{\mathrm{d}y}\Big(T(x,y)\cdot i\big(\varphi(x,y),\vartheta(x,y)\big)\Big) \qquad (6)$$

and therefore

$$\frac{\mathrm{d}I(x,y)}{\mathrm{d}y} = \frac{\mathrm{d}T(x,y)}{\mathrm{d}y}\cdot i(\varphi,\vartheta) + T(x,y)\cdot\frac{\mathrm{d}i(\varphi,\vartheta)}{\mathrm{d}y} \qquad (7)$$

Generally, we have $T(x, y) > 0$ and $i(\varphi, \vartheta) > 0$. Hence, in order for the first term in Eq. (7) to compensate the second term at least in part, we should have, in the center x0, y0 of measuring region 12, i.e. at $x = x0$ and $y = y0$:

$$\mathrm{d}T(x,y)/\mathrm{d}y > 0 \text{ and } \mathrm{d}i(\varphi(x,y),\vartheta(x,y))/\mathrm{d}y < 0 \text{ or} \qquad (8a)$$

$$\mathrm{d}T(x,y)/\mathrm{d}y < 0 \text{ and } \mathrm{d}i(\varphi(x,y),\vartheta(x,y))/\mathrm{d}y > 0 \qquad (8b)$$

The optimum location for e.g. *y*0 is where the derivative of Eq. (7) is zero, i.e.

$$\frac{\mathrm{d}T(x,y)}{\mathrm{d}y}\cdot i(\varphi,\vartheta) + T(x,y)\cdot\frac{\mathrm{d}i(\varphi,\vartheta)}{\mathrm{d}y} = 0 \qquad (9)$$

In practice, an exact equality as given in Eq. (9) is not required. If we assume $\Delta y$ to be half the extension of the measuring region along y, i.e. *H/2* as mentioned above, we advantageously should have at location $x = x0$ and $y = y0$, i.e. at the location where the optical axis 40 intersects the measuring region:

$$\Delta y\left|\frac{1}{T(x,y)}\frac{\mathrm{d}T(x,y)}{\mathrm{d}y} + \frac{1}{i(\varphi(x,y),\vartheta(x,y))}\frac{\mathrm{d}i(\varphi(x,y),\vartheta(x,y))}{\mathrm{d}y}\right| < t \qquad (10)$$

with the threshold t being no more than than 0.2, in particular smaller than 0.1.

In another approach, the measuring region is such that the irradiance $I(x,y)$ over all coordinates x, y in the measurement region varies no more than 20%, in particular no more than 10%.

For a paraboloid mirror, such as shown in Fig. 8, a sufficiently uniform irradiance can be achieved over a given height $\Delta y$ of the measurement region by choosing a sufficiently large focal length *f*. If *I*max designates the maximum intensity in the measurement region, *I*min the minimum desired intensity in the measurement region (with *I*min/*I*max > 0.8, in particular > 0.9), the coordinates $r/f = R1$ and $R2$ are determined where $T(r/f)\cdot i(r/f) = 0.8$ or 0.9, respectively. From this, and since $R1$ and $R2$ are in units of *f*, we have $f = 2 * \Delta y / (R2 - R1)$.

In this context, the extension $\Delta y$ is, e.g., defined as the region where the elongate object 10 is at least 90% of the time during the measurement.

Hence, in a preferred aspect, the invention relates to a sensor where condition (10) is maintained.

*Detector Design*

Advantageously, detector mirror 44 has parabolic or paraboloid shape as well in order to concentrate the incoming light onto detector 24. Its axis is parallel to axis 50 of collimator mirror 34.

The center of detector 24 is located at the focal point of this parabolic or paraboloid shape at a focal distance f from its apex. Hence, the beams propagating along output optical axis 40 are focused on detector 24.

However, detector mirror 44 can also be any other type of convex mirror able to send the light from measuring region 12 onto the detector's active surface.

Also, as shown in Fig. 2 and 4, there is no reason to mutually align detector mirror 44 and detector 24 such that the condition corresponding to relation (9) above is true unless the detector has angle-dependent sensitivity. Advantageously, detector mirror 44 deflects light coinciding with output optical axis 40 by 90°, in particular within +/- 5° in order to achieve a compact and efficient design.

Alternatively, detector mirror 44 can be dispensed with, e.g. when focusing the light from measuring region 12 onto detector 24 by means of a lens or when using a detector that is sufficiently large to receive all light from measuring region 12 directly.

*Notes*

In the examples above, elongate object 10 is a yarn. It must be noted, though, that it may also be another object, such as a yarn precursor, a wire, a ply yarn, or a fiber.

Elongate object 10 is advantageously moving through measuring region 12 in a direction corresponding to its longitudinal axis 11.

In the figures, optical axis 40 and carrier plate 16 are parallel. It must be noted, though, that there can also be a non-zero angle between them if the mirrors are adjusted accordingly.

While there are shown and described presently preferred embodiments of the invention, it is to be distinctly understood that the invention is not limited thereto but may be otherwise variously embodied and practiced within the scope of the following claims.

**Claims**

1. A sensor for measuring at least one property of an elongate body, in particular an elongate textile body, comprising

   a measuring region (12),
   a divergent, anisotropic light source (22) having a main emission direction (23),
   a collimator (36) arranged in a light path between said light source (22) and said measuring region (12), wherein said collimator (36) has an input optical axis (38) intersecting a center (22a) of said light source (22) and an output optical axis (40) and is adapted to project light from said light source (22) on said input optical axis (38) into said output optical axis (40), wherein the output optical axis (40) crosses a center of the measuring region (12) and wherein said collimator comprises a collimation mirror (34),
   a light detector (24) receiving light emitted by said collimator (36) and transmitted through said measuring region (12),
   a first transparent element (14a) having an input side (28a), an output side (28b), and a mirror surface (30) forming the collimation mirror (34), wherein the sensor is adapted such that light from said light source (22) enters said first transparent element (14a) through an input surface (31a) at said input side (28a), is reflected at said mirror surface (30), and exits said first transparent element (14a) through an output surface (31b) at said output side (28b) to enter said measuring region (12), wherein said input side (28a) and said output side (28b) are transversal to each other,
   wherein said input optical axis (38) and said output optical axis (40) are non-parallel, and said main emission direction (23) is non-parallel to said input optical axis (38),
   **characterized in that** said input surface (31a) is located in a concave recess (32) of the first transparent element (14a) at the input side (28a).

2. The sensor of claim 1 wherein the sensor is adapted such that, at least in a plane of said input and output optical axes (38, 40),

   a light beam emitted from the center (22a) of said light source (22) having an angle $\varphi$ in respect to said main emission direction (23) transverses the measuring region (12) at coordinates x, y,
   and wherein an irradiance $I(x,y)$ at $x$, $y$ in said measuring region (12) is given by

   $$I(x, y) \sim T(x, y) \cdot i(\varphi(x, y), \vartheta(x, y))$$

   with

   - $x$ being a coordinate perpendicular to the input and output optical axes (38, 40), with $x = x0$ at a location of said optical axis,
   - $y$ being a coordinate perpendicular to said output optical axis (40) and parallel to the plane of the input and output optical axes (38, 40), with $y = y0$ at a location of said optical axis,
- $T(x, y)$ being a transmission function of said collimator (36), and
- $i(\varphi, \vartheta)$ being the radiant intensity of said light source (22),

wherein at $x = x0$ and $y = y0$:

$$\mathrm{d}T(x,y)/\mathrm{d}y > 0 \text{ and } \mathrm{d}i(\varphi(x,y), \vartheta(x,y))/\mathrm{d}y < 0$$

or

$$\mathrm{d}T(x,y)/\mathrm{d}y < 0 \text{ and } \mathrm{d}i(\varphi(x,y), \vartheta(x,y))/\mathrm{d}y > 0$$

3. The sensor of claim 2 wherein, at $x = x0$ and $y = y0$,

$$\Delta y \left| \frac{1}{T(x,y)} \frac{\mathrm{d}T(x,y)}{\mathrm{d}y} + \frac{1}{i(\varphi(x,y), \vartheta(x,y))} \frac{\mathrm{d}i(\varphi(x,y), \vartheta(x,y))}{\mathrm{d}y} \right| < t$$

with $t$ being a threshold of no more than 0.2, in particular of 0.1 and with $\Delta y$ being half of an extension of the measuring region (12) along y.

4. The sensor of any of the preceding claims adapted such that the irradiance in said measurement region varies by no more than 20%, in particular by no more than 10%.

5. The sensor of any of the preceding claims wherein said collimation mirror (34) is concave, in particular strictly concave, at a location (P2) where the collimation mirror (34) and the input optical axis (38) intersect.

6. The sensor of any of the preceding claims wherein, at a region where the input optical axis (38) intersects said collimation mirror (34), said collimation mirror (34) has parabolic or paraboloid shape, wherein the center (22a) of said light source (22) is arranged in a focal line or point of said parabolic or paraboloid shape.

7. The sensor of claim 6, wherein, given a ratio $R$ with

$$R = r0/f$$

with r0 being a distance between the center (22a) of said light source (22) and said output optical axis (40) and $f$ a focal length of said parabolic or paraboloid shape, said ratio $R$ is between 0.5 and 1.4, in particular between 0.7 and 1.1, in particular between 0.8 and 1.0.

8. The sensor of any of the claims 6 or 7 wherein an intersection point (P1) between said main emission direction (23) and said parabolic or paraboloid shape is further away from the center (22a) of said light source (22) than said location (P2).

9. The sensor of any of the preceding claims wherein a deflection angle ($\beta$) from said input optical axis (38) to said output optical axis (40) is smaller than 90°, in particular smaller than 80° and/or larger than 30°.

10. The sensor of any of the preceding claims comprising a carrier plate (16) arranged at said input side (28a), wherein said light source (22) is mounted to said carrier plate (16), wherein said carrier plate (16) is arranged transversally to said output surface (31b),
and in particular wherein an angle between said carrier plate (16) and said output surface (31b) is between 70° and 110°.

11. The sensor of claim 10 wherein the main emission direction (23) of said light source (22) extends perpendicular to said carrier plate (16).

12. The sensor of any of the preceding claims particular wherein at least in a plane intersecting the center (22a) of said light source (22) and being parallel to said input and output optical axes (38, 40), said concave recess (32) has a circular cross section with the center (22a) of the light source (22) being at a center of said circular cross section.

13. The sensor of any of the preceding claims comprising a solid, transparent filler material extending from a surface of said light source (22) to said input side (28a).

14. The sensor of any of the preceding claims comprising a detector mirror (44) receiving said light from the measuring region (12) and deflecting said light towards said detector (24),
and in particular wherein said detector mirror is adapted to deflect light coinciding with the output optical axis (40) by 90°, in particular within +/- 5°.

15. The sensor of claim 14 further comprising a second transparent element (14b) having an input side (41a), a mirror surface (42), and an output side (41b), wherein said mirror surface (42) forms said detector mirror (44).

16. The sensor claim 15

wherein said first and said second transparent elements (14a, 14b) are integrally connected to each other to form a transparent body (14), and/or
wherein said measuring region (12) is located between the output side (28b) of said first transparent element (14a) and the input side (41a) of said second transparent element (14b), and/or
wherein, at said measuring region (12), the output side (28b) of said first transparent element (14a) is parallel, in particular within +/- 10°, to the input side (41a) of said second transparent element (14b), and/or
wherein said sensor comprises a carrier plate (16), with said light source (22) and said light detector (24) mounted to said carrier plate (16), wherein the input side (28a) of said first transparent element (14a) and the output side (41b) of said second transparent element (14b) both abut against said carrier plate (16).

17. The sensor of any of the preceding claims wherein said detector (24) is arranged at a distance (r0) from said output optical axis (40),
and in particular wherein said detector (24) and said light source (22) and are both mounted to a common carrier plate (16).

18. Use of the sensor of any of the preceding claims for measuring a diameter of said elongate body.

**Patentansprüche**

1. Ein Sensor zur Messung mindestens einer Eigenschaft eines langgestreckten Körpers, insbesondere eines langgestreckten Textilkörpers, umfassend

einen Messbereich (12),
eine divergente, anisotrope Lichtquelle (22) mit einer Hauptemissionsrichtung (23),
einen Kollimator (36), der in einem Lichtweg zwischen der Lichtquelle (22) und dem Messbereich (12) angeordnet ist, wobei der Kollimator (36) eine optische Eingangsachse (38), die ein Zentrum (22a) der Lichtquelle (22) schneidet, und eine optische Ausgangsachse (40) aufweist und dazu ausgelegt ist, Licht von der Lichtquelle (22) auf der optischen Eingangsachse (38) in die optische Ausgangsachse (40) zu projizieren, wobei die optische Ausgangsachse (40) ein Zentrum des Messbereichs (12) kreuzt und wobei der Kollimator einen Kollimationsspiegel (34) umfasst,
einen Lichtdetektor (24), der das von dem Kollimator (36) emittierte und durch den Messbereich (12) übertragene Licht empfängt,
ein erstes transparentes Element (14a) mit einer Eingangsseite (28a), einer Ausgangsseite (28b) und einer Spiegelfläche (30), die den Kollimationsspiegel (34) bildet, wobei der Sensor so eingerichtet ist, dass Licht von der Lichtquelle (22) durch eine Eingangsfläche (31a) an der Eingangsseite (28a) in das erste transparente Element (14a) eintritt, an der Spiegelfläche (30) reflektiert wird und das erste transparente Element (14a) durch eine Ausgangsfläche (31b) an der Ausgangsseite (28b) verlässt, um in den Messbereich (12) einzutreten, wobei die Eingangsseite (28a) und die Ausgangsseite (28b) quer zueinander liegen,
wobei die optische Eingangsachse (38) und die optische Ausgangsachse (40) nicht parallel sind und die Hauptemissionsrichtung (23) nicht parallel zu der optischen Eingangsachse (38) ist,

**dadurch gekennzeichnet, dass** die Eingangsfläche (31a) in einer konkaven Ausnehmung (32) des ersten transparenten Elements (14a) an der Eingangsseite (28a) angeordnet ist.

2. Der Sensor nach Anspruch 1, wobei der Sensor so ausgebildet ist, dass zumindest in einer Ebene der optischen Eingangs- und Ausgangsachsen (38, 40),

ein vom Zentrum (22a) der Lichtquelle (22) emittierter Lichtstrahl mit einem Winkel $\varphi$ in Bezug auf die Hauptemissionsrichtung (23) den Messbereich (12) bei den Koordinaten x, y durchquert,
und wobei eine Bestrahlungsstärke I(x,y) bei x, y in dem Messbereich (12) gegeben ist durch

$$I(x,y) ~ T(x,y) \cdot i(\varphi(x,y), \vartheta(x,y))$$

wobei

- x eine Koordinate senkrecht zu den optischen Eingangs- und Ausgangsachsen (38, 40) ist, wobei x = x0 an einer Stelle der optischen Achse ist,
- y eine Koordinate senkrecht zur optischen Ausgangsachse (40) und parallel zur Ebene der optischen Eingangs- und Ausgangsachsen (38, 40) ist, wobei y = y0 an einem Ort der optischen Achse ist,
- T(x, y) eine Übertragungsfunktion des Kollimators (36) ist, und
- i($\varphi$, $\vartheta$) die Strahlungsintensität der Lichtquelle (22) ist,

wobei bei x = x0 und y = y0:

$$dT(x,y)/dy>0 \text{ und } di(\varphi(x,y),\vartheta(x,y))/dy<0$$

oder

$$dT(x,y)/dy<0 \text{ und } di(\varphi(x,y),\vartheta(x,y))/dy>0$$

3. Der Sensor nach Anspruch 2, wobei bei x = x0 und y = y0,

$$\Delta y \left| \frac{1}{T(x,y)} \frac{dT(x,y)}{dy} + \frac{1}{i(\varphi(x,y),\vartheta(x,y))} \frac{di(\varphi(x,y),\vartheta(x,y))}{dy} \right| < t$$

wobei t ein Schwellenwert von höchstens 0,2, insbesondere von 0,1 ist und $\Delta y$ die Hälfte einer Ausdehnung der Messregion (12) entlang y ist.

4. Der Sensor nach einem der vorangehenden Ansprüche, der so ausgestaltet ist, dass die Bestrahlungsstärke im Messbereich um nicht mehr als 20 %, insbesondere um nicht mehr als 10 %, schwankt.

5. Der Sensor nach einem der vorangehenden Ansprüche, wobei der Kollimationsspiegel (34) an einer Stelle (P2), an der sich der Kollimationsspiegel (34) und die optische Eingangsachse (38) schneiden, konkav, insbesondere streng konkav, ist.

6. Der Sensor nach einem der vorangehenden Ansprüche, wobei der Kollimationsspiegel (34) in einem Bereich, in dem die optische Eingangsachse (38) den Kollimationsspiegel (34) schneidet, eine Parabel- oder Paraboloidform aufweist, wobei das Zentrum (22a) der Lichtquelle (22) in einer Brennlinie oder einem Punkt der Parabel- oder Paraboloidform angeordnet ist.

7. Der Sensor nach Anspruch 6, wobei bei einem Verhältnis R mit

$$R = r0/f$$

wobei r0 ein Abstand zwischen dem Zentrum (22a) der Lichtquelle (22) und der optischen Ausgangsachse (40) und f eine Brennweite der Parabel- oder Paraboloidform ist, das Verhältnis R zwischen 0,5 und 1,4, insbesondere zwischen 0,7 und 1,1, insbesondere zwischen 0,8 und 1,0 liegt.

8. Der Sensor nach einem der Ansprüche 6 oder 7, wobei ein Schnittpunkt (P1) zwischen der Hauptemissionsrichtung (23) und der Parabel- oder Paraboloidform weiter vom Zentrum (22a) der Lichtquelle (22) entfernt ist als die Stelle (P2).

9. Der Sensor nach einem der vorangehenden Ansprüche, wobei ein Ablenkungswinkel (β) von der optischen Eingangsachse (38) zur optischen Ausgangsachse (40) kleiner als 90° ist, insbesondere kleiner als 80° und/oder grösser als 30°.

10. Der Sensor nach einem der vorangehenden Ansprüche, umfassend eine an der Eingangsseite (28a) angeordnete Trägerplatte (16), wobei die Lichtquelle (22) an der Trägerplatte (16) angebracht ist, wobei die Trägerplatte (16) quer zur Ausgangsfläche (31b) angeordnet ist,
und wobei insbesondere ein Winkel zwischen der Trägerplatte (16) und der Ausgangsfläche (31b) zwischen 70° und 110° beträgt.

11. Der Sensor nach Anspruch 10, wobei die Hauptemissionsrichtung (23) der Lichtquelle (22) senkrecht zur Trägerplatte (16) verläuft.

12. Der Sensor nach einem der vorangehenden Ansprüche, insbesondere bei dem die konkave Aussparung (32) zumindest in einer Ebene, die den Mittelpunkt (22a) der Lichtquelle (22) schneidet und parallel zu den optischen Eingangs- und Ausgangsachsen (38, 40) verläuft, einen kreisförmigen Querschnitt aufweist, wobei der Mittelpunkt (22a) der Lichtquelle (22) in der Mitte des kreisförmigen Querschnitts liegt.

13. Der Sensor nach einem der vorangehenden Ansprüche umfassend ein festes, transparentes Füllmaterial, das sich von einer Oberfläche der Lichtquelle (22) zur Eingangsseite (28a) erstreckt.

14. Der Sensor nach einem der vorangehenden Ansprüche, umfassend einen Detektorspiegel (44), der das Licht aus dem Messbereich (12) empfängt und das Licht zum Detektor (24) umlenkt,
und insbesondere wobei der Detektorspiegel geeignet ist, das mit der optischen Ausgangsachse (40) zusammenfallende Licht um 90°, insbesondere innerhalb von +/- 5°, abzulenken.

15. Der Sensor nach Anspruch 14, weiter umfassend ein zweites transparentes Element (14b) mit einer Eingangsseite (41a), einer Spiegelfläche (42) und einer Ausgangsseite (41b), wobei die Spiegelfläche (42) den Detektorspiegel (44) bildet.

16. Der Sensor nach Anspruch 15,

wobei das erste und das zweite transparente Element (14a, 14b) einstückig miteinander verbunden sind, um einen transparenten Körper (14) zu bilden, und/oder
wobei der Messbereich (12) zwischen der Ausgangsseite (28b) des ersten transparenten Elements (14a) und der Eingangsseite (41a) des zweiten transparenten Elements (14b) angeordnet ist, und/oder
wobei im Messbereich (12) die Ausgangsseite (28b) des ersten transparenten Elements (14a) parallel, insbesondere innerhalb von +/- 10°, zu der Eingangsseite (41a) des zweiten transparenten Elements (14b) ist, und/oder
wobei der Sensor eine Trägerplatte (16) umfasst, wobei die Lichtquelle (22) und der Lichtdetektor (24) an der Trägerplatte (16) angebracht sind, wobei die Eingangsseite (28a) des ersten transparenten Elements (14a) und die Ausgangsseite (41b) des zweiten transparenten Elements (14b) beide an der Trägerplatte (16) anliegen.

17. Der Sensor nach einem der vorangehenden Ansprüche, wobei der Detektor (24) in einem Abstand (r0) von der optischen Ausgangsachse (40) angeordnet ist,
und insbesondere der Detektor (24) und die Lichtquelle (22) beide an einer gemeinsamen Trägerplatte (16) befestigt sind.

18. Verwendung des Sensors nach einem der vorhergehenden Ansprüche zur Messung eines Durchmessers des langgestreckten Körpers.

**Revendications**

1. Un capteur pour mesurer au moins une propriété d'un corps allongé, en particulier d'un corps textile allongé, comprenant

une zone de mesure (12),
une source de lumière anisotrope divergente (22) ayant une direction d'émission principale (23),
un collimateur (36) disposé dans un trajet optique entre la source de lumière (22) et la zone de mesure (12),
le collimateur (36) ayant un axe optique d'entrée (38) qui coupe un centre (22a) de la source de lumière (22) et un axe optique de sortie (40) et étant adapté à projeter de la lumière provenant de la source de lumière (22) sur l'axe optique d'entrée (38) dans l'axe optique de sortie (40), l'axe optique de sortie (40) croisant un centre de la zone de mesure (12), et dans lequel le collimateur comprend un miroir de collimation (34),
un détecteur de lumière (24) qui reçoit la lumière émise par le collimateur (36) et transmise à travers la zone de mesure (12),
un premier élément transparent (14a) ayant un côté d'entrée (28a), un côté de sortie (28b) et une surface de miroir (30) formant le miroir de collimation (34), le détecteur étant agencé de sorte que la lumière provenant de la source de lumière (22) entre dans le premier élément transparent (14a) par une surface d'entrée (31a) au niveau du côté d'entrée (28a), est réfléchie sur la surface de miroir (30) et quitte le premier élément transparent (14a) par une surface de sortie (31b) sur le côté de sortie (28b) pour entrer dans la zone de mesure (12), le côté d'entrée (28a) et le côté de sortie (28b) étant transversaux l'un par rapport à l'autre,
dans lequel l'axe optique d'entrée (38) et l'axe optique de sortie (40) ne sont pas parallèles et la direction d'émission principale (23) n'est pas parallèle à l'axe optique d'entrée (38),
**caractérisé en ce que** la surface d'entrée (31a) est disposée dans un évidement concave (32) du premier élément transparent (14a) sur le côté d'entrée (28a) .

2. Le capteur selon la revendication 1, dans lequel le capteur est configuré de telle sorte qu'au moins dans un plan des axes optiques d'entrée et de sortie (38, 40),

un faisceau de lumière émis par le centre (22a) de la source de lumière (22) avec un angle $\varphi$ par rapport à la direction principale d'émission (23) traverse la zone de mesure (12) aux coordonnées x, y,
et dans lequel une intensité de rayonnement I(x,y) en x, y dans la zone de mesure (12) est donnée par

$$I(x,y) \sim T(x,y) \cdot i(\varphi(x,y), \vartheta(x,y))$$

où

- x est une coordonnée perpendiculaire aux axes optiques d'entrée et de sortie (38, 40), où x = x0 en un point de l'axe optique,
- y est une coordonnée perpendiculaire à l'axe optique de sortie (40) et parallèle au plan des axes optiques d'entrée et de sortie (38, 40), où y = y0 en un point de l'axe optique,
- T(x, y) est une fonction de transfert du collimateur (36), et
- i($\varphi$, $\vartheta$) est l'intensité du rayonnement de la source lumineuse (22),

où, pour x = x0 et y = y0 :

$$dT(x,y)/dy>0 \text{ et } di(\varphi(x,y), \vartheta(x,y))/dy<0$$

ou

$$dT(x,y)/dy<0 \text{ et } di(\varphi(x,y), \vartheta(x,y))/dy>0$$

3. Le capteur selon la revendication 2, où pour x = x0 et y = y0,

$$\Delta y \left| \frac{1}{T(x,y)} \frac{\mathrm{d}T(x,y)}{\mathrm{d}y} + \frac{1}{i(\varphi(x,y), \vartheta(x,y))} \frac{\mathrm{d}i(\varphi(x,y), \vartheta(x,y))}{\mathrm{d}y} \right| < t$$

où t est une valeur seuil d'au plus 0,2, en particulier de 0,1, et $\Delta y$ est la moitié d'une extension de la région de mesure (12) le long de y.

4. Le capteur selon l'une des revendications précédentes, qui est configuré de telle sorte que l'intensité du rayonnement ne varie pas de plus de 20 %, notamment de plus de 10 %, dans la zone de mesure.

5. Le capteur selon l'une des revendications précédentes, dans lequel le miroir de collimation (34) est concave, notamment strictement concave, en un point (P2) où le miroir de collimation (34) et l'axe optique d'entrée (38) se croisent.

6. Le capteur selon l'une des revendications précédentes, dans lequel le miroir de collimation (34) présente une forme parabolique ou paraboloïde dans une zone où l'axe optique d'entrée (38) coupe le miroir de collimation (34), le centre (22a) de la source de lumière (22) étant situé dans une ligne focale ou un point de la forme parabolique ou paraboloïde.

7. Le capteur selon la revendication 6, dans lequel, pour un rapport R avec

```
R = r0/f
```

où r0 est une distance entre le centre (22a) de la source de lumière (22) et l'axe optique de sortie (40) et f est une distance focale de la forme parabolique ou paraboloïde, le rapport R est compris entre 0,5 et 1,4, notamment entre 0,7 et 1,1, en particulier entre 0,8 et 1,0.

8. Le capteur selon l'une des revendications 6 ou 7, dans lequel un point d'intersection (P1) entre la direction d'émission principale (23) et la forme parabolique ou paraboloïde est plus éloigné du centre (22a) de la source de lumière (22) que le point (P2).

9. Le capteur selon l'une des revendications précédentes, dans lequel un angle de déviation ($\beta$) de l'axe optique d'entrée (38) par rapport à l'axe optique de sortie (40) est inférieur à 90°, notamment inférieur à 80° et/ou supérieur à 30°.

10. Le capteur selon l'une des revendications précédentes, comprenant une plaque de support (16) disposée sur la surface d'entrée (28a), la source de lumière (22) étant montée sur la plaque de support (16), la plaque de support (16) étant disposée transversalement à la surface de sortie (31b),
et dans lequel, en particulier, un angle entre la plaque de support (16) et la surface de sortie (31b) est compris entre 70° et 110°.

11. Le capteur selon la revendication 10, dans lequel la direction d'émission principale (23) de la source de lumière (22) est perpendiculaire à la plaque de support (16).

12. Le capteur selon l'une des revendications précédentes, en particulier dans lequel l'évidement concave (32) présente une section transversale circulaire au moins dans un plan sécant au centre (22a) de la source de lumière (22) et parallèle aux axes optiques d'entrée et de sortie (38, 40), le centre (22a) de la source de lumière (22) étant situé au milieu de la section transversale circulaire.

13. Le capteur selon l'une des revendications précédentes, comprenant un matériau de remplissage solide et transparent s'étendant depuis une surface de la source de lumière (22) jusqu'au côté d'entrée (28a).

14. Le capteur selon l'une des revendications précédentes, comprenant un miroir de détection (44) recevant la lumière de la zone de mesure (12) et redirigeant la lumière vers le détecteur (24),
et en particulier dans lequel le miroir de détection est apte à dévier la lumière coïncidant avec l'axe optique de sortie (40) de 90°, notamment à +/- 5°.

15. Le capteur selon la revendication 14, comprenant en outre un deuxième élément transparent (14b) ayant une surface d'entrée (41a), une surface de miroir (42) et une surface de sortie (41b), la surface de miroir (42) formant le miroir de détection (44).

16. Le détecteur selon la revendication 15,

dans lequel les premier et deuxième éléments transparents (14a, 14b) sont reliés entre eux en une pièce pour former un corps transparent (14), et/ou

la zone de mesure (12) étant située entre la surface de sortie (28b) du premier élément transparent (14a) et la surface d'entrée (41a) du deuxième élément transparent (14b), et/ou

dans lequel, dans la zone de mesure (12), le côté de sortie (28b) du premier élément transparent (14a) est parallèle, en particulier à +/- 10°, au côté d'entrée (41a) du deuxième élément transparent (14b), et/ou

le capteur comprenant une plaque de support (16), la source de lumière (22) et le détecteur de lumière (24) étant montés sur la plaque de support (16), le côté d'entrée (28a) du premier élément transparent (14a) et le côté de sortie (41b) du deuxième élément transparent (14b) étant tous deux en contact avec la plaque de support (16).

17. Le capteur selon l'une des revendications précédentes, dans lequel le détecteur (24) est situé à une distance (r0) de l'axe optique de sortie (40),

et en particulier le détecteur (24) et la source de lumière (22) sont tous deux fixés à une plaque de support commune (16).

18. Utilisation du capteur selon l'une des revendications précédentes pour mesurer un diamètre du corps allongé.

**Fig. 1**

**Fig. 2**

**Fig. 3**

**Fig. 4**

**Fig. 5**

**Fig. 6**

**Fig. 7**

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2827127 A

- US 47616942 B